# EUROPÄISCHE PATENTSCHRIFT

(11) **EP 1 306 057 B1**
(45) Veröffentlichungstag und Bekanntmachung des Hinweises auf die Patenterteilung: **02.11.2005**
(21) Anmeldenummer: 02018890.0
(22) Anmeldetag: 23.08.2002
(51) Int. Cl.: A61B 17/80

(54) **Knochenfixationseinrichtung**
Bones fixation device
Dispositif de fixation d'os

(30) Priorität: 23.10.2001 DE 10152094
(43) Veröffentlichungstag der Anmeldung: 02.05.2003
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE); Crockard, Alan, c/o Dsc, FRCS, Queen Square, London WC1N 3BG (GB)
(74) Vertreter: Prüfer, Lutz H.

(56) Entgegenhaltungen:
- EP-A- 0 897 697
- EP-A- 0 903 113
- WO-A-96/05778
- CH-A- 672 245
- FR-A- 2 726 461
- US-A- 5 735 853

## Beschreibung

Die Erfindung betrifft eine Knochenfixationseinrichtung und in einer besonderen Anwendung eine Einrichtung zur Stabilisierung von Halswirbeln.

Es ist eine Einrichtung insbesondere für die Stabilisierung von Halswirbeln bekannt, die eine Platte mit wenigstens einer ein M-Gewinde aufweisenden Bohrung und eine Knochenschraube zum Einschrauben in die Platte umfasst. Die Knochenschraube weist einen ein Knochengewinde aufweisenden Gewindeabschnitt zum Einschrauben in den Knochen und einen daran anschließenden Gewindeabschnitt mit einem M-Außengewinde auf. Im Betrieb wird die Knochenschraube durch die Bohrung hindurchgeführt und in den Knochen eingeschraubt. Dann wird der zweite Gewindeabschnitt in die Platte eingeschraubt. Auf Grund der unterschiedlichen Gewindesteigungen besteht die Gefahr, daß die Knochenschraube dadurch aus ihrem Sitz gelöst wird.

Aus der WO 96/05778 ist ein Halswirbelstabilisierungssystem bekannt, welches eine Platte mit wenigstens einer Ausnehmung, eine Knochenschraube mit einem Knochengewindeabschnitt und einem daran anschließenden M-Gewindeabschnitt und einem zwischen dem Knochengewindeabschnitt und dem M-Gewindeabschnitt vorgesehenen radialen Ansatz sowie eine mit dem M-Gewindeabschnitt zusammenwirkende Mutter aufweist. Bei dieser bekannten Einrichtung wird zuerst die Knochenschraube in den Knochen eingeschraubt, dann die Platte mit der Ausnehmung über den M-Gewindeabschnitt geführt, bis sie an dem radialen Ansatz anliegt und anschließend die Mutter in die Ausnehmung eingeführt und auf den M-Gewindeabschnitt zum Fixieren aufgeschraubt. Die Ausnehmung kann als Langloch ausgebildet sein.

Aus der WO 00/18312 ist eine Stabilisierungseinrichtung für die Wirbelsäule bekannt, die eine Platte mit wenigstens einer Ausnehmung aufweist, eine Knochenschraube mit einem Knochengewindeabschnitt und einem M-Gewindeabschnitt und einem zwischen diesen Abschnitten vorgesehenen Ansatz und mit einer Unterlegscheibe, die auf dem Rand der Ausnehmung aufliegt, sowie einer Mutter, die über den M-Gewindeabschnitt der Knochenschraube aufgeschraubt wird, bis sie an der Beilagscheibe anliegt. Die Unterlegscheibe hat eine seitliche Ausnehmung, dass die Knochenschraube verschiedene Winkelstellungen einnehmen kann.

Die bekannten Systeme sind jeweils für bestimmte Anwendungen geeignet. So muss bei der zuerst genannten Einrichtung immer zuerst die Platte angelegt werden und anschließend die Schraube eingeschraubt werden. Bei der aus der WO 96/05778 bekannten Einrichtung muss hingegen zuerst die Schraube eingeschraubt werden und dann die Platte aufgelegt werden. Ferner ist mit dieser Einrichtung keine Änderung der Winkelstellung der Schraube möglich. Die aus der WO 00/18312 bekannte Einrichtung erlaubt zwar unterschiedliche Winkelstellungen der Knochenschraube, eine Justierung in Längsrichtung der Platte ist jedoch nur begrenzt möglich. Ferner hat diese Einrichtung einen relativ hohen Aufbau.

Aus der EP 0 897 697 A1 (Basis für den Oberbegriff des Anspruchs 1) ist eine Fixationseinrichtung für Knochen mit einer Platte mit wenigstens einer Ausnehmung zum Hindurchführen eines Abschnitts einer Knochenschraube und einem in der Ausnehmung verschiebbaren Verbindungselement zum Verbinden der Knochenschraube mit der Platte bekannt. Das Verbindungselement ist dadurch am Herausfallen gesichert, daß es im wesentlichen hülsenförmig ausgebildet ist und eine ringförmige Ausnehmung besitzt, durch die ringförmige Vorsprünge gebildet sind, die aufgrund von Schlitzen in der Wandung komprimierbar sind. Beim Einführen des Verbindungselements werden die Vorsprünge zusammengedrückt. Wenn das Verbindungselement an der richtigen Stelle sitzt, rasten die Vorsprünge in entsprechende Ausnehmungen in der Platte ein, so daß das Verbindungselement durch die Öffnung nicht mehr herausfallen kann.

Es ist Aufgabe der Erfindung, eine Knochenfixationseinrichtung und insbesondere eine Einrichtung zur Halswirbelstabilisierung bereitzustellen, die bezüglich der Justierbarkeit und der Anwendungsbreite gegenüber den bekannten Einrichtungen verbessert ist.

Diese Aufgabe wird gelöst durch eine Fixationseinrichtung für Knochen gemäß Patentanspruch 1 und ein Fixationssystem mit einer solchen Fixationseinrichtung nach Patentanspruch 10. Weiterbildungen der Erfindung sind in den abhängigen Ansprüchen angegeben.

Die Erfindung weist den besonderen Vorteil eines modularen Systems auf, welches die Anwendungsbreite wesentlich vergrößert. Die erfindungsgemäße Vorrichtung ist nicht nur zur Halswirbelstabilisierung einsetzbar, sondern auch als Knochenfixationseinrichtung zum Fixieren von gebrochenen Knochen, z.B. von Röhrenknochen. Die Erfindung ermöglicht es ferner, daß der Chirurg im Operationssaal, angepaßt an die Umstände des Einzelfalls eine Entscheidung treffen kann, ob zuerst die Schrauben einzubringen sind und anschließend die Platte darübergelegt und fixiert wird, oder ob die Platte angelegt werden soll und dann die Knochenschrauben durch die Ausnehmungen der Platte hindurch einzuschrauben sind. Ferner erlaubt es die Erfindung zusammen mit einer Platte Monoaxial- und Polyaxialschrauben oder sogar beide Schraubentypen nebeneinander zu verwenden. Damit ist die Justierung der Winkelstellung der Schrauben zur Platte und die Positionierung der Schrauben zur Justierung ihres Längsabstandes für jede Schraube unabhängig möglich.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine perspektivische Ansicht der Knochenfixationseinrichtung nach einer ersten Ausführungsform;
- Fig. 2: eine Draufsicht auf die Platte der Knochenfixationseinrichtung gemäß Fig. 1;
- Fig. 3: eine perspektivische Explosionsdarstellung der Knochenschraube und der Unterlegscheibe der Knochenfixationseinrichtung gemäß Fig. 1;
- Fig. 4: einen Schnitt durch einen Teil der Knochenfixationseinrichtung gemäß Fig. 1;
- Fig. 5: eine perspektivischen Explosionsdarstellung einer Knochenfixationseinrichtung nach einer zweiten Ausführungsform;
- Fig. 6: einen Schnitt durch die Knochenfixationseinrichtung gemäß der zweiten Ausführungsform;
- Fig. 7: eine perspektivische Explosionsdarstellung einer Knochenfixationseinrichtung gemäß einer dritten Ausführungsform;
- Fig. 8: einen Schnitt durch die Knochenfixationseinrichtung der dritten Ausführungsform;
- Fig. 9: eine perspektivische Explosionsdarstellung einer Knochenfixationseinrichtung gemäß einer vierten Ausführungsform;
- Fig. 10: einen Schnitt durch die Knochenfixationseinrichtung gemäß der vierten Ausführungsform;
- Fig. 11: eine perspektivische Darstellung einer Abwandlung der Knochenschraube der vorhergehenden Ausführungsformen;
- Fig. 12: eine abgewandelte Form der Platte der Kinochenfixationseinrichtung der vorhergehenden Ausführungsformen.

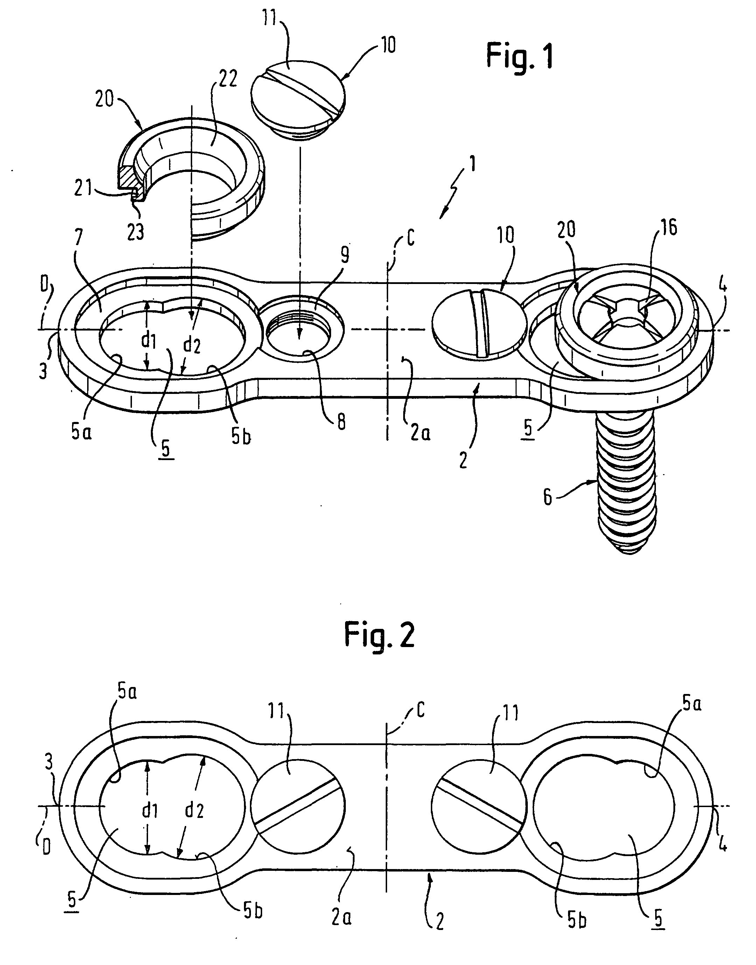

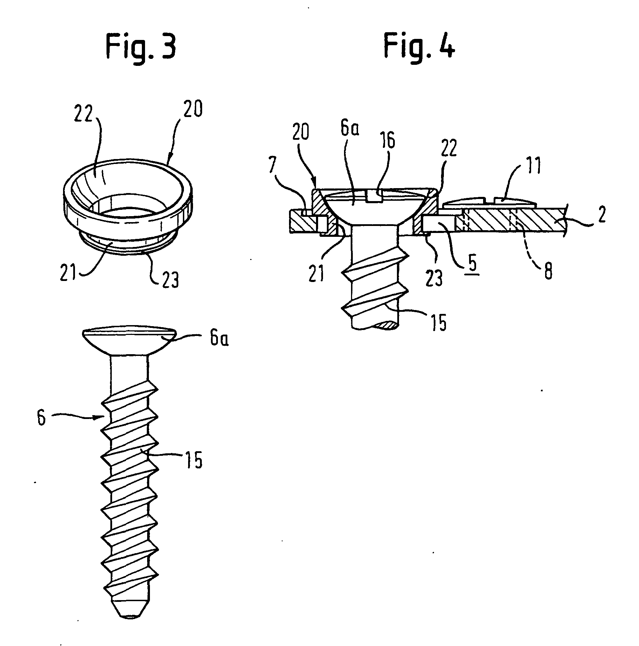

Wie aus den Figuren 1 bis 4 ersichtlich ist, weist die Knochenfixationseinrichtung 1 eine im wesentlichen rechteckige Platte 2 mit abgerundeten Ecken auf. Für die weitere Beschreibung wird die in Längsrichtung des Rechtecks verlaufende Mittenlinie der Platte mit D und die senkrecht dazu in Querrichtung verlaufende Mittenlinie mit C bezeichnet. Die Platte weist ein erstes Ende 3 und ein diesem gegenüberliegendes zweites Ende 4 mit jeweils nahe den Enden vorgesehenen Ausnehmungen 5 zur Aufnahme von Knochenschrauben auf. Jede Ausnehmung 5 weist einen ersten Bereich 5a mit einem ersten Durchmesser d₁ in einer Richtung senkrecht zur Mittenlinie D und einen zweiten Bereich 5b mit einem zweiten Durchmesser d₂ in einer Richtung senkrecht zur Mittenlinie D auf, wobei der zweite Durchmesser d₂ größer ist als der erste Durchmesser d₁. Der gesamte Durchmesser der Ausnehmung 5 in Längsrichtung, also auf der Mittenlinie D ist größer als der zweite Durchmesser d₂. Die Bereiche 5a und 5b sind beispielsweise durch ein Langloch, dessen kleiner Durchmesser dem Durchmesser des Bereichs 5a entspricht, und einer zusätzlichen Bohrung mit dem größeren Durchmesser d₂ des Bereichs 5b gebildet. Die Bereiche 5a und 5b sind auf der Platte derart angeordnet, dass der Bereich 5b dem jeweiligen Ende 3 bzw. 4 der Platte abgewandt ist und zur Mittenlinie C zeigt. An der im Gebrauchszustand dem Schaft der Knochenschraube 6 abgewandten Oberseite 2a der Platte 2 weist jede Ausnehmung 5 eine Senkung 7 zur Aufnahme eines nachfolgend beschriebenen Verbindungselements auf.

Die Platte 2 weist ferner angrenzend an die Bereiche 5b der Ausnehmungen 5 einander gegenüberliegende Gewindebohrungen 8 mit einer Senkung 9 zum Einschraubenen von Schrauben 10 mit Kopf 11, die beispielsweise als Schlitzschrauben ausgebildet sind, auf. Die Gewindebohrung 8 ist in einem solchen Abstand zu dem Bereich 5b der Ausnehmung 5 vorgesehen und die Senkung 9 sowie der Kopf 11 der Schraube 10 sind so dimensioniert, dass bei eingeschraubtem Zustand der Kopf 11 gerade ein wenig in den Bereich der Senkung 7 im Bereich 5b der Ausnehmung 5 hineinragt und so den Querschnitt der Senkung 7 beschränkt.

Wie insbesondere aus den Figuren 1, 3 und 4 ersichtlich ist, weist die Knochenfixationseinrichtung zum Verbinden der Knochenschraube mit der Platte 2 ein Verbindungselement in Form eines hülsenartig ausgebildeten Unterlegelements bzw. Formstücks, im folgenden Unterlegscheibe 20 genannt, auf. Die Unterlegscheibe 20 weist einen hohlzylindrischen Abschnitt 21 auf, dessen Länge etwas größer ist, als die Dicke der Platte im Bereich der Ausnehmung 5. An dem in die Platte 2 eingesetzten Zustand an der Oberseite 2a der Platte angeordneten Seite ist die Unterlegscheibe 20 mit einem hohlkugelsegmentförmigen Abschnitt 22 ausgebildet, dessen Außendurchmesser kleiner ist als der Durchmesser der Senkung 7 quer zur Mittenlinie D, aber größer als der Durchmesser d₂ des zweiten Bereichs 5b, so dass die Unterlegscheibe 20 mit dem Bereich 22 in eingesetztem Zustand am Rand der Senkung 7 aufliegt. Der hohlkugelsegmentförmige Abschnitt 22 ist an seiner Innenseite mit einem Kugelradius ausgebildet, der dem Kugelradius eines kugelsegmentförmigen Kopfes 6a der Knochenschraube 6 entspricht. An dem dem hohlkugelsegmentförmigen Abschnitt 22 abgewandten Ende weist die Unterlegscheibe 20 einen ringförmigen Ansatz 23 auf, dessen Außendurchmesser größer ist, als der Durchmesser d₁ des ersten Bereichs 5a der Ausnehmung 5 und kleiner als der Durchmesser d₂ des zweiten Bereichs 5b der Ausnehmung 5. Somit ist die Unterlegscheibe 20 so dimensioniert, daß sie durch den Bereich 5b mit dem größeren Durchmesser d₂ der Ausnehmung 5 in die Ausnehmung 5 einsetzbar ist und in Längsrichtung der Platte in der Senkung 7 bis in den Bereich 5a verschiebbar ist, aus dem sie nicht herausfallen kann. Die Senkung 7 bildet eine Führung für die Unterlegscheibe.

Die Knochenfixationseinrichtung 1 der ersten Ausführungsform weist ferner die Knochenschraube 6 auf, die in dieser Ausführungsform einstückig ausgebildet ist mit einem Schaft 15 mit einem Knochengewinde zum Einschrauben in den Knochen und mit dem mit dem Schaft festverbundenen kugelsegmentförmigen Kopf 6a, der an seiner Oberseite ein Mittel 16, z.B. einen oder mehrere Schlitze, zum in Eingriff bringen mit einem Eindrehwerkzeug aufweist.

Im Betrieb wird zuerst die Unterlegscheibe 20 durch den Bereich 5b der Ausnehmung 5 eingeführt, so daß sich der hohlkugelsegmentförmige Abschnitt 22 an der Oberseite 2a der Platte befindet. Anschließend wird die Unterlegscheibe in Richtung des Bereiches 5a verschoben, wobei die Unterseite des hohlkugelsegmentförmigen Abschnitts 22 in der Senkung 7 aufliegt. In dem Bereich 5a mit dem kleineren Durchmesser d₁ ist die Unterlegscheibe 20 aufgrund der größeren Durchmesser des hohlkugelsegmentförmigen Abschnitts 22 und des Ansatzes 23 gegen Herausfallen gehalten. Anschließend wird die Sicherungsschraube 9 in die Bohrung 8 eingeschraubt. Der Rand des Kopfes 11 der Sicherungsschraube 9 ragt dabei, wie insbesondere aus den Figuren 1, 2 und 4 ersichtlich ist, ein wenig in den Bereich der Senkung 7 der Ausnehmung 5 hinein und beschränkt somit den Durchmesser der Senkung 7 in dem Bereich 5b, wodurch die Unterlegscheibe 20 nach ihrem Einsetzen gegen Herausfallen gesichert ist. Dabei ist jedoch die Unterlegscheibe 20 noch in der Ausnehmung 5 in Längsrichtung verschiebbar und wird an die gewünschte exakte Stelle geschoben. Anschließend werden die Knochenschrauben 6 mit dem Gewindeschaft 15 mit dem Knochengewinde durch die Unterlegscheibe hindurchgeführt und in den Knochen eingeschraubt.

Im Operationsbetrieb verwendet der Chirurg die Platte mit bereits eingesetzten und gegen Herausfallen durch die Schrauben 9 gesicherten Unterlegscheiben 20, die somit jeweils ein integriertes Formstück bilden, und hält diese an die zu fixierenden Knochenteile an und schraubt anschließend die Knochenschrauben 6 ein. Durch die Verschiebbarkeit der Unterlegscheibe ist eine Justierung der Schraubposition vor dem Einschrauben möglich.

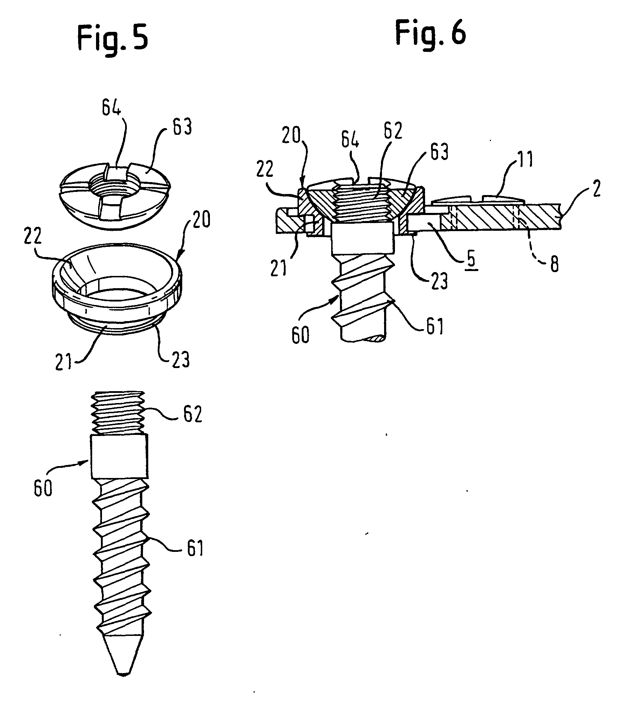

Die in den Figuren 5 und 6 gezeigte Ausführungsform ist, was die Platte 2 anbetrifft, identisch zu der in den Figuren 1 bis 4 gezeigten Ausführungsform. Die Knochenschraube 60 ist jedoch bei dieser Ausführungsform verschieden. Die Knochenschraube 60 weist einen ersten Gewindeschaftabschnitt 61 mit einem Knochengewinde und einen daran angrenzenden zweiten Gewindeschaftabschnitt 62 mit einem M-Gewinde auf. Der Durchmesser des zweiten Gewindabeschnitts 62 ist kleiner als der Innendurchmesser der Unterlegscheibe 20, so daß der zweite Gewindeabschnitt 62 durch die hülsenartig gebildete Unterlegscheibe 20 hindurchführbar ist. An ihrem der Spitze der Knochenschraube gegenüberliegenden Ende weist die Knochenschraube eine nicht dargestellte Sechskantausnehmung zum in Eingriff bringen mit einem Eindrehwerkzeug zum Einschrauben der Knochenschraube in den Knochen auf.

Der Kopf der Schraube 60 wird bei dieser Ausführungsform durch eine separate Mutter 63 gebildet, die kugelsegmentförmig mit einem Radius, der dem des hohlkugelsegmentförmigen Abschnitts 22 der Unterlegscheibe 20 entspricht, geformt ist. Die Mutter 63 weist an ihrer dem kugelsegmentförmigen Teil abgewandten Oberseite ein Mittel 64, wie z.B. Schlitze, zum in Eingriff bringen mit einem Eindrehwerkzeug auf.

Im Betrieb wird wiederum zuerst die Unterlegscheibe bzw. die Unterlegscheiben 20 in die Ausnehmungen 5 der Platte 2 eingeführt und gegen Herausfallen über die Schraube 10 gesichert. Bei der Operation wird zuerst die Platte 2 an die zu fixierenden Knochenteile angehalten und die Einschraubposition für die Knochenschrauben markiert. Anschließend werden die Knochenschrauben 60 eingeschraubt und die Platte 2 mit den Ausnehmungen 5 über den aus dem Knochen hervorstehenden zweiten Gewindeabschnitt 62 gesetzt. Dann wird die Mutter 63 auf dem zweiten Gewindeabeschnitt aufgeschraubt bis sie in dem hohlkugelsegmentförmigen Abschnitt 22 der Unterlegscheibe 20 zu liegen kommt, wobei aufgrund der sphärischen Abschnitte der Unterlegscheibe 20 und der Mutter 63 Winkelstellungen der Knochenschraube in einem vorbestimmten Winkelbereich möglich sind.

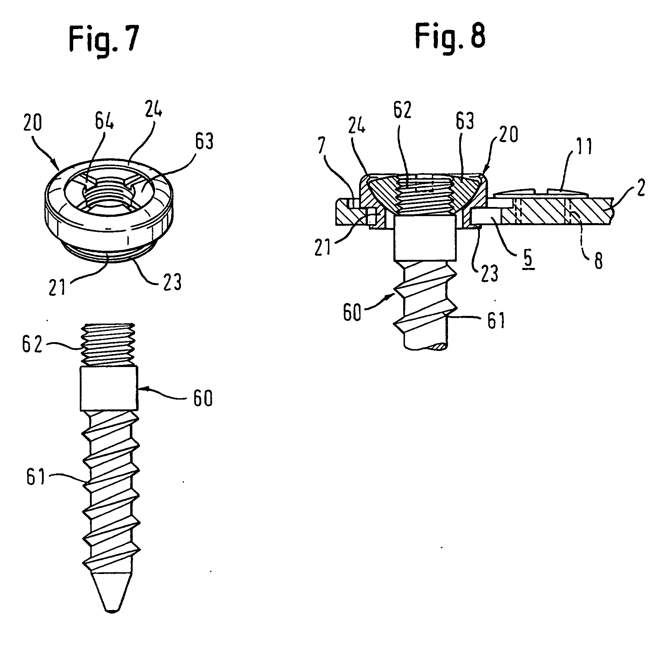

Die in den Figuren 7 und 8 gezeigte Ausführungsform unterscheidet sich von der in den Figuren 5 und 6 gezeigten Ausführungsformen dadurch, daß die Unterlegscheibe 20 am äußeren Ende des hohlkugelsegmentförmigen Abschnitts 22 einen um ein vorbestimmtes Maß nach innen ragenden Rand 24 aufweist, der die eingesetzte Mutter 63 von ihrer Oberseite her um ein vorbestimmtes Maß umgreift, so daß die Mutter 63 gegen Herausfallen gesichert ist.

Im Betrieb ist bei dieser Ausführungsform die Unterlegscheibe 20 mit bereits eingesetzter Mutter 63 vorgefertigt und wird wie bei den zuvor beschriebenen Ausführungsformen erst in die Platte 2 eingesetzt und mit der Sicherungsschraube 10 gegen Herausfallen gesichert. Der weitere Betrieb ist wie bei der in den Figuren 5 und 6 gezeigten Ausführungsform. Diese Ausführungsform hat den Vorteil, daß die Mutter 63 gehalten wird und somit dem Chirurgen bei der Operation nicht entfallen kann.

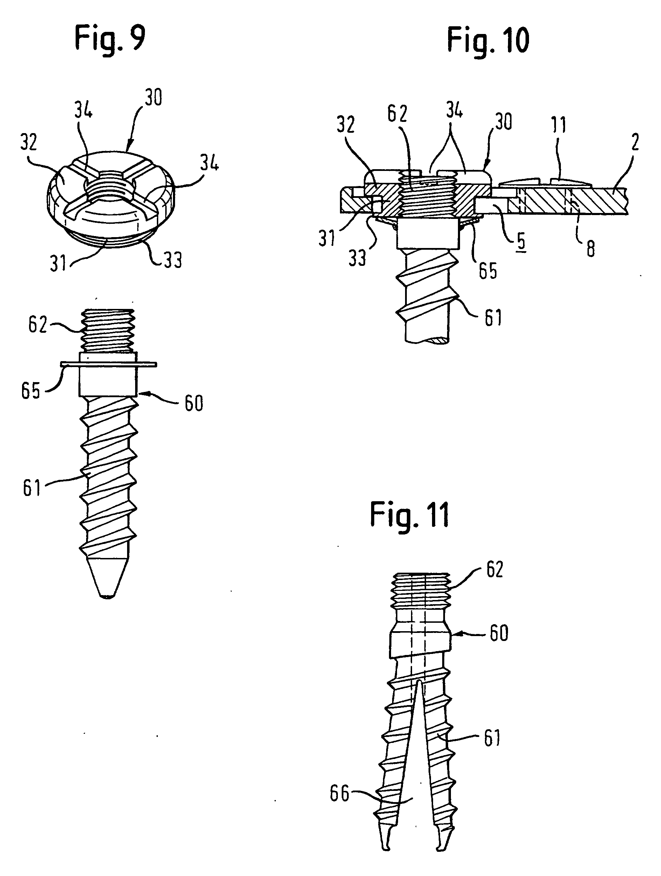

Bei der in Figuren 9 und 10 gezeigten Ausführungsform ist das Verbindungselement zwischen der Platte und der Knochenschraube anstelle als Unterlegscheibe als Mutter 30 zum Aufschrauben auf den zweiten Gewindeschaftabschnitt 62 der Knochenschraube 60 ausgebildet. Die Mutter 30 weist einen ersten Bereich 31 auf, der in seinen Abmessungen dem hohlzylindrischen Mittelteil 21 der Unterlegscheibe der vorherigen Ausführungsformen entspricht und aber ein Innengewinde aufweist. Angrenzend an das eine Ende dieses Mittenabschnitts weist die Mutter 30 einen Kopfabschnitt 32 mit einem Außendurchmesser auf, der wie bei der Unterlegscheibe 20 etwas kleiner als der Durchmesser der Senkung 7 aber größer als der Durchmesser d₂ des zweiten Bereichs 5b der Ausnehmung 5 ist. Angrenzend an das andere Ende des Mittenbereichs 31 ist wie bei der Unterlegscheibe ein ringförmiger Ansatz 33 vorgesehen. Die Mutter 30 weist ferner an ihrer Oberseite ebenfalls Mittel 34, wie z.B. Schlitze zum in Eingriff bringen mit einem Eindrehwerkzeug auf.

Die Knochenschraube 60 ist bei dieser Ausführungsform mit einer zwischen dem Knochengewindeschaftabschnitt 61 und dem Gewindeschaftbeschnitt 62 mit dem M-Gewinde angeordneten Schulter 65 versehen, die beispielsweise als ringförmiger Vorsprung gebildet ist und als Anschlag beim Einschrauben dient, wie insbesondere in Fig. 10 dargestellt ist.

Im Betrieb wird an den zuvor bestimmten Positionen die Knochenschraube bzw. die Knochenschrauben 60 eingeschraubt, anschließend die Platte mit den Ausnehmungen 5 über die Gewindeabschnitte 62 aufgelegt und dann die Mutter 30 aufgeschraubt.

Bei der in Fig. 11 gezeigten Ausführungsform ist die Knochenschraube des Knochengewindeschaftabschnitts hohl ausgebildet und mit zwei gegenüberliegenden Längsschlitzen 66 versehen. Es ist ferner ein in der Figur nicht dargestelltes Spreizelement im Inneren des Schafts vorgesehen, welches derart betätigbar ist, daß die durch die Längsschlitze 66 gebildeten Schaftabschnitte auseinanderspreizbar sind. Nach dem Einschrauben des Knochengewindeschaftabschnitts wird somit eine Spreizwirkung erzielt, die einen verbesserten Halt gewährleistet.

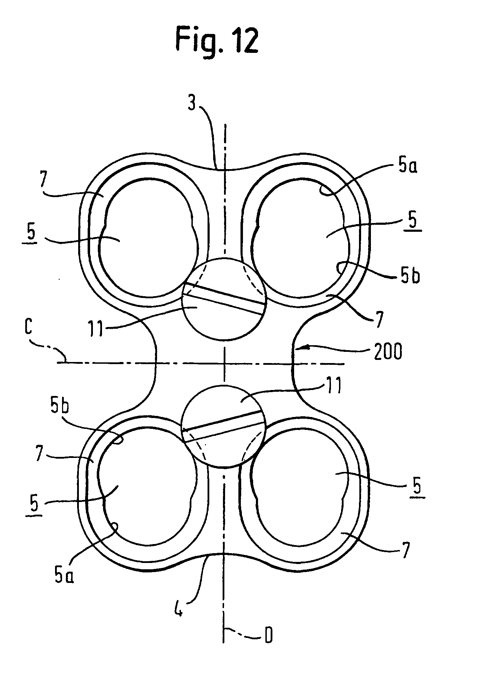

Bei der in Fig. 12 dargestellten Ausführungsform ist die Platte 200 insbesondere zur Anwendung bei der Halswirbelstabilisierung ausgebildet und weist an jedem Ende 3, 4 jeweils zwei Ausnehmungen 5 auf, wobei sich jeweils zwei benachbarte Ausnehmungen 5 eine Sicherungsschraube 10 teilen. Dazu ist die Achse der Bohrung 8 für die Sicherungsschraube 10 auf der Mittenlängsachse D angeordnet und die Dimension des Schraubenkopfes 11 und die Position der Bohrung ist so gewählt, daß der Schraubenkopf 11 in eingeschraubtem Zustand in beide Senkungen 7 der benachbarten Ausnehmungen 5 eingreift. Ferner ist die Platte 200 tailliert ausgebildet.

Die beschriebenen Ausführungsformen können auf einer Platte beliebig miteinander kombiniert werden oder die Schrauben können, je nach Zweck, für die verschiedenen Ausführungsformen verwendet werden, was ein modulares System mit einer großen Vielseitigkeit gewährleistet.

Anstelle der Sicherungschraube 10 kann auch ein anderes Sicherungselement vorgesehen sein, beispielsweise ein an der Platte angebrachtes Federelement, das zum Einsetzen der Unterlegscheibe 20 oder der der Mutter 30 weggebogen wird und dann zum Begrenzen des Querschnitts der Ausnehmung zurückschnappt.

Der Begriff Platte ist so zu verstehen, daß darunter auch Elemente mit einer nicht ganz ebenen, beispielsweise mit einer gekrümmten oder anderweitig gebogenen Oberfläche fallen.

Die erfindungsgemäße Vorrichtung läßt sich nicht nur auf die Stabilisierung von Halswirbel anwenden, sondern ist auch geeignet für die externe Fixierung von gebrochenen Knochen, z. B. von Röhrenknochen.

## Patentansprüche

1. Fixationseinrichtung für Knochen mit
einer Platte (2, 200) mit wenigstens einer Ausnehmung (5) zum Hindurchführen eines Abschnittes (15, 61, 62) einer Knochenschraube (6, 60),
einem in der Ausnehmung (5) verschiebbaren Verbindungselement (20, 30) zum Verbinden der Knochenschraube mit der Platte und einer Sicherungseinrichtung (10, 11) zum Sichern des Verbindungselements in der Ausnehmung, **dadurch gekennzeichnet, daß** die Ausnehmung (5) einen ersten Bereich (5a) aufweist in dem das Verbindungselement (20, 30) gehalten ist, und in der Plattenoberfläche benachbart zu dem ersten Bereich (5a) einen zweiten Bereich (5b), der einen größeren Durchmesser als der erste Bereich aufweist, wobei der zweite Bereich so dimensioniert ist, daß das Verbindungselement in die Ausnehmung einführbar ist.

2. Fixationseinrichtung nach Anspruch 1, wobei die Sicherungseinrichtung ein Sicherungselement (10) umfaßt, das den Querschnitt des zweiten Bereichs begrenzt.

3. Fixationseinrichtung nach Anspruch 1 oder 2, **dadurch gekennzeichnet, daß** das Verbindungselement (20, 30) hülsenförmig ausgebildet ist zur Aufnahme des der Spitze abgewandten Endes der Knochenschraube.

4. Fixationseinrichtung nach Anspruch 3, **dadurch gekennzeichnet, daß** das eine Ende der Hülse einen Ansatz (23, 33) aufweist, dessen größter Durchmesser größer als der größte Durchmesser des ersten Bereichs (5a) der Ausnehmung (5) und kleiner als der größte Durchmesser des zweiten Bereichs (5b) ist und dessen größter Außendurchmesser an dem dem Ansatz gegenüberliegenden Ende größer ist als der größte Durchmesser des zweiten Bereichs (5b).

5. Fixationseinrichtung nach Anspruch 3 oder 4, **dadurch gekennzeichnet, daß** die Länge der Hülse (20, 30) geringfügig größer als die Dicke der Platte (2, 200) am Rand der Ausnehmung (5) ist.

6. Fixationseinrichtung nach einem der Ansprüche 1 bis 5, **dadurch gekennzeichnet, daß** die Sicherungseinrichtung eine in die Platte (2, 200) einschraubbare Schraube (10) umfaßt die so angeordnet ist, daß in eingeschraubtem Zustand ihr Kopf (11) in den zweiten Bereich (5b) der Ausnehmung soweit hineinragt, daß er ein Hindurchführen des Verbindungselements (20, 30) verhindert.

7. Fixationseinrichtung nach einem der Ansprüche 1 bis 6, **dadurch gekennzeichnet, daß** die Ausnehmung (5) der Platte eine Senkung aufweist.

8. Fixationseinrichtung nach einem der Ansprüche 1 bis 7, **dadurch gekennzeichnet daß** die Platte (200) jeweils zwei nebeneinanderliegende Ausnehmungen (5) aufweist und die Sicherungseinrichtung (10, 11) für beide Ausnehmungen gemeinsam vorgesehen ist.

9. Fixationseinrichtung nach einem der Ansprüche 1 bis 8, **dadurch gekennzeichnet, daß** das Verbindungselement als Unterlegelement (20) mit einem hohlkugelsegmentförmigen Abschnitt (22) zur Aufnahme eines kugelsegmentförmigen Kopfes (6a, 63) der Knochenschraube (6, 60) ausgebildet ist.

10. Fixationssystem mit einer Fixationseinrichtung nach einem der Ansprüche 1 bis 9 und einer in die Ausnehmung (5) einführbaren Knochenschraube (6), **dadurch gekennzeichnet, daß** der Kopf der Schraube als kugelsegmentförmige Mutter (63) ausgebildet ist.

11. Fixationssystem nach Anspruch 10, **dadurch gekennzeichnet, daß** der hohlkugelsegmentförmige Abschnitt (22, 24) des Unterlegelements (20) die Mutter (63) derart umgreift, daß diese in dem hohlkugelsegmentförmigen Abschnitt eingeschlossen ist.

12. Fixationssystem nach einem der Ansprüche 10 bis 11, **dadurch gekennzeichnet, daß** das Verbindungselement als Mutter (30) ausgebildet ist, welche den Kopf der Knochenschraube bildet.

13. Fixationssystem nach Anspruch 10, **dadurch gekennzeichnet, daß** die Knochenschraube (6) einen Abschnitt (15) mit einem Knochengewinde und einen integrierten Kopf (6a) aufweist.

14. Fixationssystem nach einem der Ansprüche 10 bis 13, **dadurch gekennzeichnet, daß** die Knochenschraube (60) einen ersten Abschnitt (61) mit einem Knochengewinde und daran anschließend einen zweiten Abschnitt (62) mit einem Gewinde zum Verbinden mit einer Mutter (63, 30) aufweist.

15. Fixationssystem nach Anspruch 14, **dadurch gekennzeichnet, daß** die Knochenschraube zwischen dem ersten und dem zweiten Abschnitt eine Schulter (65) aufweist.

16. Fixationssystem nach einem der Ansprüche 10 bis 15, **dadurch gekennzeichnet, daß** das in den Knochen einzuschraubende Ende der Knochenschraube (60) geschlitzt ist.

## Claims

1. Fixing device for bones with
a plate (2, 200) with at least one recess (5) for guiding-through a section (15, 61, 62) of a bone screw (6, 60),
a connecting element (20, 30), displaceable in the recess (5), for connecting the bone screw to the plate, and
a securing device (10, 11) for securing the connecting element in the recess;
**characterized in that**
the recess (5) has a first area (5a) in which the connecting element (20, 30) is held and, in the plate surface adjacent to the first area (5a), a second area (5b), which has a larger diameter than the first area, wherein the second area is dimensioned such that the connecting element is insertable into the recess.

2. Fixing device according to claim 1, wherein the securing device comprises a securing element (10) which limits the cross-section of the second area.

3. Fixing device according to claim 1 or 2, **characterised in that** the connecting element (20, 30) is constructed as sleeve-shaped for receiving the end of the bone screw facing away from the tip.

4. Fixing device according to claim 3, **characterised in that** one end of the sleeve has a projection (23, 33), the largest diameter of which is larger than the largest diameter of the first area (5a) of the recess (5) and smaller than the largest diameter of the second area (5b) and the largest outer diameter of which at the end opposite the projection is larger than the largest diameter of the second area (5b).

5. Fixing device according to claim 3 or 4, **characterised in that** the length of the sleeve (20, 30) is slightly larger than the thickness of the plate (2, 200) at the edge of the recess (5).

6. Fixing device according to one of claims 1 to 5, **characterised in that** the securing device comprises a screw (10) screwable into the plate (2, 200) which is arranged such that in the screwed-in state its head (11) projects into the second area (5b) of the recess to such an extent that it prevents guiding-through of the connecting element (20, 30).

7. Fixing device according to one of claims 1 to 6, **characterized in that** the recess (5) of the plate has an indentation.

8. Fixing device according to one of claims 1 to 7, **characterised in that** the plate (200) has two recesses (5) respectively located next to one another and the securing device (10, 11) is provided for both recesses jointly.

9. Fixing device according to one of claims 1 to 8, **characterised in that** the connecting element is constructed as a washer element (20) with a hollow spherical segment shaped section (22) for receiving a spherical segment shaped head (6a, 63) of the bone screw (6, 60).

10. Fixing system with a fixing device according to one of claims 1 to 9 and with a bone screw (6) insertable into the recess (5) **characterised in that** the head of the screw is constructed as a spherical segment shaped nut (63).

11. Fixing system according to claim 10, **characterised in that** the hollow spherical segment shaped section (22, 24) of the washer element (20) encompasses the nut (63) in such a way that it is enclosed in the hollow spherical segment shaped section.

12. Fixing system according to one of claims 10 to 11, **characterised in that** the connecting element is constructed as a nut (30) that forms the head of the bone screw.

13. Fixing system according to claim 10, **characterised in that** the bone screw (6) has a section (15) with a bone thread and an integrated head (6a).

14. Fixing system according to one of claims 10 to 13, **characterised in that** the bone screw (60) has a first section (61) with a bone thread and adjoining it a second section (62) with a thread for connecting to a nut (63, 30).

15. Fixing system according to claim 14, **characterised in that** the bone screw has a shoulder (65) between the first and the second section.

16. Fixing device according to one of claims 10 to 15, **characterised in that** the end of the bone screw (60) to be screwed into the bone is slit.

## Revendications

1. Dispositif de fixation pour os, avec
une plaque (2, 200) avec au moins un creux (5) pour le passage d'une section (15, 61, 62) d'une vis pour os (6, 60),
avec uri élément de liaison (20, 30) déplaçable dans le creux (5) pour relier la vis pour os avec la plaque et avec un dispositif de blocage (10, 11) pour bloquer l'élément de liaison dans le creux, **caractérisé en ce que** le creux (5) comporte une première région (5a), dans laquelle l'élément de liaison (20, 30) est maintenu et, dans la surface de la plaque, dans le voisinage de la première région (5a), une deuxième région (5b), dont le diamètre est supérieur à celui de la première région, la deuxième région étant dimensionnée de façon à ce que l'élément de liaison puisse être introduit dans le creux.

2. Dispositif de fixation selon la revendication 1, le dispositif de blocage comportant un élément de blocage (10) qui délimite la section transversale de la deuxième région.

3. Dispositif de fixation selon la revendication 1 ou 2, **caractérisé en ce que** l'élément de liaison (20, 30) est conçu sous forme de douille, pour loger l'extrémité de la vis pour os qui est opposée à la pointe.

4. Dispositif de fixation selon la revendication 3, **caractérisé en ce que** l'une des extrémités de la douille comporte un tenon (23, 33) dont le diamètre le plus grand est supérieur au diamètre le plus grand de la première région (5a) du creux (5) et inférieur au diamètre le plus grand de la deuxième région (5b) et dont le diamètre extérieur le plus grand sur l'extrémité opposée au tenon est supérieur au plus grand diamètre de la deuxième région (5b).

5. Dispositif de fixation selon la revendication 3 ou 4, **caractérisé en ce que** la longueur de la douille (20, 30) est légèrement supérieure à la grosseur de la plaque (2, 200) sur le bord du creux (5).

6. Dispositif de fixation selon l'une quelconque des revendications 1 à 5, **caractérisé en ce que** le dispositif de blocage comprend une vis (10) destinée à être vissée dans la plaque (2, 200), qui est disposée de façon à ce qu'à l'état vissé, sa tête (11) saillisse assez loin dans la deuxième région (5b) du creux, pour éviter un passage de l'élément de liaison (20, 30).

7. Dispositif de fixation selon l'une quelconque des revendications 1 à 6, **caractérisé en ce que** le creux (5) de la plaque comporte un renfoncement.

8. Dispositif de fixation selon l'une quelconque des revendications 1 à 7, **caractérisé en ce que** la plaque (200) comporte respectivement deux creux (5) disposés côte à côte et **en ce que** le dispositif de blocage (10, 11) est prévu en commun pour les deux creux.

9. Dispositif de fixation selon l'une quelconque des revendications 1 à 8, **caractérisé en ce que** l'élément de liaison est conçu en tant qu'élément d'insertion (20) avec une section en forme de segment sphérique creux (22) pour réceptionner une tête en forme de segment sphérique (6a, 63) de la vis pour os (6, 60).

10. Système de fixation avec un dispositif de fixation selon l'une quelconque des revendications 1 à 9 et avec une vis à os (6) pouvant être introduite dans le creux (5), **caractérisé en ce que** la tête de la vis est conçue en tant qu'écrou en forme de segment sphérique (63).

11. Système de fixation selon la revendication 10, **caractérisé en ce que** la section en forme de segment sphérique creux (22, 24) de l'élément d'insertion (20) entoure l'écrou (63) de façon à ce que celui-ci soit enfermé dans la section en forme de segment sphérique creux.

12. Système de fixation selon l'une quelconque des revendications 10 à 11, **caractérisé en ce que** l'élément de liaison est conçu en tant qu'écrou (30) qui forme la tête de la vis pour os.

13. Système de fixation selon la revendication 10, **caractérisé en ce que** la vis pour os (6) comporte une section (15) avec un filetage pour os et une tête intégrée (6a).

14. Système de fixation selon l'une quelconque des revendications 10 à 13, **caractérisé en ce que** la vis pour os (60) comporte une première section (61) avec un filetage pour os et une deuxième section (62) qui s'y raccorde avec un filetage destiné à être relié à un écrou (63, 30).

15. Système de fixation selon la revendication 14, **caractérisé en ce que** la vis pour os comporte un épaulement (65) entre la première et la deuxième section.

16. Système de fixation selon l'une quelconque des revendications 10 à 15, **caractérisé en ce que** l'extrémité de la vis pour os (60) destinée à être vissée dans l'os est fendue.
